Europäisches Patentamt

European Patent Office

Office européen des brevets

(19)

(11) Publication number: **0 193 637**
**A1**

# (12) EUROPEAN PATENT APPLICATION

(21) Application number: **85102670.8**

(22) Date of filing: **08.03.85**

(51) Int. Cl.⁴: **A 61 K 31/535**

(43) Date of publication of application: **10.09.86**
**Bulletin 86/37**

(84) Designated Contracting States: **BE CH DE FR GB IT LI NL SE**

(71) Applicant: **Yoshitomi Pharmaceutical Industries, Ltd.,**
**35 Hiranomachi 3-chome Higashi-ku, Osaka-shi**
**Osaka 541 (JP)**

(72) Inventor: **Setoguchi, Michihide,**
**5-21 Chuo-machi 2-Chome, Nakatsu Oita 871 (JP)**
Inventor: **Anami, Koretake, 49-4 Takase-Hayashida,**
**Nakatsu Oita 871 (JP)**
Inventor: **Yasuda, Hiroshi, 5-16 Chuo-machi 2-Chome,**
**Nakatsu Oita 871 (JP)**

(74) Representative: **von Kreisler, Alek, Dipl.-Chem. et al,**
**Deichmannhaus am Hauptbahnhof, D-5000 Köln 1 (DE)**

(54) Use for the improvement or treatment of various diseases associated with cerebrovascular damages.

(57) A use of 2-(2-(2-thienylmethyl)phenoxymethyl)morpholine or a pharmaceutically acceptable acid addition salt thereof for the manufacture of a medicament for the improvement or treatment of various diseases associated with cerebrovascular damages or aging, is disclosed.

EP 0 193 637 A1

SPECIFICATION


Field of the Invention


The present invention relates to a new use of
2-(2-(2-thienylmethyl)phenoxymethyl)morpholine or a
pharmaceutically acceptable acid addition salt thereof.


Background of the Invention


(1)  U. S. Patent No. 4005084 or G. B. Patent No. 1466820
discloses 2-(2-(2-thienylmethyl)phenoxymethyl)morpholine or a
pharmaceutically acceptable acid addition salt thereof having
an anti-ulcer activity.

(2)  Severe diseases caused by various cerebrovascular
damages or aging have been increasing as the social tentions
become higher.   Furthermore, the increase of aged population
accelerates such kind of diseases.

Therefore, there have been strong demands for the development
of the drugs or methods to improve or treat such diseases.

Under these circumstances, many efforts have been performed
to obtain effective drugs or establish therapeutical methods
for various diseases associated with cerebrovascular damages
or aging.

Disclosure of the Invention


The present inventors have also made intensive investigations,
and now have found that 2-(2-(2-thienylmethyl)phenoxymethyl)-

morpholine or a pharmaceutically acceptable acid addition salt thereof is remarkably effective compound for the improvement or treatment of such diseases.

The present invention relates to a use of 2-(2-(2-thienylmethyl)phenoxymethyl)morpholine or a pharmaceutically acceptable acid addition salt thereof for the manufacture of a medicament for the improvement of treatment of various diseases associated with cerebrovascular damages or aging.

The pharmaceutically acceptable acid addition salt includes, for example, maleate, fumarate, succinate, citrate, acetate, malate, benzoate, hydrochloride, hydrobromide, phosphate and sulfate.

Since the 2-(2-(2-thienylmethyl)phenoxymethyl)morpholine compound has an asymmetric carbon on the morpholine nucleus, there exist racemate and individual optical isomers. The present invention embrances them.

As a result of a series of pharmacological experiments by the present inventors, it is established that 2-(2-(2-thienylmethyl)phenoxymethyl)morpholine or the pharmaceutically acceptable acid addition salt thereof exhibits anti-amnesic activity, anti-hypoxic activity, anti-anoxic activity, reparative activity on cerebral energy state and increasing activity on glucose uptake into brain, and that the compounds are useful as an active ingredient of the drugs for the improvement or treatment of various diseases associated with cerebrovascular damages or aging.

Various diseases to be improved or treated by means of the present invention include, for example, senile dimentia, Alzheimer disease, and such symptoms as amnesia, disorientation, reduced initiation, reduced motivation, emotional disturbance and depressive state and so on caused by aging, head injury, brain operation, cerebral apoplexy, cerebral infarction, cerebral hemorrhage, cerebral athrosclerosis or cerebral tumor.

The following pharmacological experiments illustrate the present invention in more detail. The compound employed in the experiments is as below.

Compound A : 2-(2-(2-thienylmethyl)phenoxymetyl)morpholine maleate.

Experiment 1 : Effects on experimentally induced amnesia for one trial passive avoidance response in mice

Male ddY-mice, weighing 19-25 g were used. The test apparatus consisted of a two-compartment avoidance box with a large 10x23x20 cm compartment illuminated by a 40W bulb, and a small 14x14x20 cm shock compartment, painted black, with a floor made of metal rods 6 mm apart through which a foot shock (FS, 0.25 mA, 2 seconds) could be delivered. The two compartments were separated by a guillotine door.

The training procedure consisted of placing the mouse in the center of the large compartment. Within 20 to 50 seconds the animal entered the small compartment and was immediately given FS, and removed from the compartment. The animals were exposed to $CO_2$ for 8 seconds or given an electroconvulsive shock (ECS, 30 mA, 0.5 seconds) immediately after FS, or were injected with an amnestic agent scopolamine (SCO, 3 mg/kg i.p.)

20 minutes before FS. ·

The testing procedure consisted of placing the animal in the large compartment as above and noting the latency to enter the dark compartment up to 600 seconds. The training-to-test interval was 24 hours. Compound A or vehicle (0.5 % methylcellulose) was injected intraperitoneally immediately after exposure to $CO_2$ or ECS. For the scopolamine induced amnesia model, they were injected intraperitoneally immediately after FS. Thirty mice were used per test group.

The results in latency (second) observed are summerized in the following Tables 1 to 3.

Table 1 : Effect of Compound A on $CO_2$-induced amnesia for step-through passive avoidance response in mice.

| | Dose (mg/kg, i.p.) | Latency[1] (sec.) |
|---|---|---|
| Control | — | 509.5 ± 29.8** |
| $CO_2$-vehicle control | — | 201  ± 30.9 |
| $CO_2$-Compound A | 5 | 324.5 ± 37.1** |

**$p < 0.01$: significant difference vs. $CO_2$-vehicle control
1): mean ± standard error

Table 2 : Effect of Compound A on electroconvulsive shock (ECS) induced amnesia for step-through passive avoidance response in mice.

0193637

| | Dose (mg/kg, i.p.) | Latency[1] (sec.) |
|---|---|---|
| Control | — | 489.8 ± 48.7** |
| ECS-vehicle cotrol | — | 220.8 ± 40.7 |
| ECS-Compound A | 5 | 357.5 ± 46.5* |

*$p < 0.05$, **$p < 0.01$: significant difference vs. ECS-vehicle control

1): mean ± standard error

Table 3 : Effect of Compound A on scopolamine induced amnesia

for step-through passive avoidance response in mice

| | Dose (mg/kg, i.p.) | Latency[1] (sec.) |
|---|---|---|
| Control | — | 486.8 ± 34.9** |
| SCO-vehicle control | — | 78.6 ± 10.9 |
| SCO-Compound A | 5 | 169.6 ± 26.7* |

*$p < 0.05$, **$p < 0.01$: significant difference vs. SCO-vehicle control

1): mean ± standard error

Experiment 2 : Effects on hypoxia

(1) Effect on normabaric hypoxia

The experiment was performed according to the method by Yasuda et al described in Arch. Int. Pharmacolodyn., vol. 233, p. 136-144 (1978).

Three male ddY-mice were introduced into a 300 ml glass container in which circulated a current of a gas mixture of nitrogen and oxygen (96:4) at the rate of about 5l/minutes.

The time to respiratory failure was recorded.

Table 4 : Effect on normabaric hypoxia

|            | Dose (mg/kg,i.p.) | Survival time[1] (min.) |
|------------|-------------------|-------------------------|
| Control    | -                 | 2.68 ± 0.17             |
| Compound A | 30                | 3.87 ± 0.32**           |

** $p < 0.01$ : significant difference vs. control
1): mean ± standard error .

(2)   Effect on hypobaric hypoxia

According to the method of Nakanishi et al described in Life Sci., vol. 13, p. 467-474 (1973), female ddY-mice were intraperitoneally injected with Compound A and 30 minutes later placed in a sealed chamber; then the pressure was decreased to 210 mmHg. The survival time, from induction of hypoxia to respiratory failure, of the animals was determined up to 15 minutes and compared with that obtained in the control group.

Table 5 : Effect on hypobaric hypoxia

|            | Dose (mg/kg,i.p.) | Survival time (sec.) log t ± S.E. |
|------------|-------------------|-----------------------------------|
| Control    | -                 | 2.43 ± 0.04                       |
| Compound A | 30                | 2.74 ± 0.06**                     |

** $p < 0.01$ : significant difference vs. control

S.E. means standard error

Experiment 3 : Effect on KCN induced anoxia

(1)　Effect on recovery of righting reflex

Male dd-mice were intraperitoneally injected with Compound A 30 minutes prior to the intravenous injection of 1.8 mg/kg of KCN-saline solution.　The time to recovery of righting reflex, coma time, was determined.

Table 6 : Effect on KCN-induced coma

| | Dose (mg/kg,i.p.) | Coma time (sec.) (log t ± S.E.) |
|---|---|---|
| Control | - | 2.16 ± 0.040 |
| Compound A | 10 | 1.95 ± 0.039** |

** $p < 0.01$ : significant difference vs. control

S.E. means standard error

(2)　Effect on KCN-induced mortality

Male dd-mice were intraperitoneally given Compound A 30 minutes before the intravenous injection of a lethal dose (2.5 mg/kg) of KCN-saline solution.　Inhibitory effect on the mortality by KCN was tested.

Table 7 : Inhibitory effect on KCN-induced mortality

| | Dose (mg/kg,i.p.) | Survival animals/ animals used | Lethality |
|---|---|---|---|
| Control | - | 2/10 | 80 |
| Compound A | 30 | 10/10** | 0 |

** $p < 0.01$ : significant difference vs. control

Experiment 4 : Effect on cerebral energy state in

KCN-induced anoxia mice

Male ddY-mice weighing 22-26 g were used.  Compound A was dissolved in 0.5 % methylcellulose solution and injected intraperitoneally.  After administration, 2.5 mg/kg of KCN in saline was injected intravenously.  The animals were frozen in liquid nitrogen at 30 or 120 seconds laterm  After removing and weighing, the frozen cerebrums were powdered. According to the procedure by Lowry, O. H. et al described in "A flexible system of enzymatic analysis" (Academic press 1978) or by Yasuda et al described in Arch. Int. Pharmacodyn., vol. 242, p. 77-85 (1979), contents of Phosphocreatine (P-creatine), ATP, ADP, AMP, glucose and lactate in brain were determined. Concentrations ($\mu$ moles/g of brain) were calculated. Student's t-test was used in the statical analysis of the results.

The results are summerized in Table 8.

Table 8 : Effect on cerebral energy state in KCN-induced anoxia mice

| | Intact | Time after KCN (2.5mg/kg i.v.) | | | |
| | | 30 sec. | | 120 sec. | |
| | | Control | Compound A | Control | Compound A |
|---|---|---|---|---|---|
| P-creatine | 3.27 ± 0.16 | 1.80 ± 0.10 | 2.52 ± 0.13** | 0.07 ± 0.03 | 2.40 ± 0.09** |
| ATP | 2.74 ± 0.12 | 2.33 ± 0.05 | 2.62 ± 0.07** | 0.28 ± 0.02 | 2.60 ± 0.02** |
| ADP | 0.496 ± 0.026 | 0.730 ± 0.011 | 0.599 ± 0.020** | 0.651 ± 0.024 | 0.509 ± 0.023** |
| AMP | 0.066 ± 0.008 | 0.219 ± 0.018 | 0.107 ± 0.009** | 2.159 ± 0.051 | 0.044 ± 0.005** |
| ECP[a] | 0.904 ± 0.003 | 0.820 ± 0.017 | 0.880 ± 0.004** | 0.197 ± 0.007 | 0.905 ± 0.004** |
| Glucose | 1.67 ± 0.16 | 0.56 ± 0.05 | 1.25 ± 0.11** | 0.14 ± 0.02 | 0.64 ± 0.04** |
| Lactate | 1.69 ± 0.19 | 5.65 ± 0.20 | 4.92 ± 0.17** | 9.90 ± 0.37 | 7.42 ± 0.42** |

Concentrations are in μmol/g of wet tissue.        ** p < 0.01 : significant difference vs. control

a) : Energy charge potential (ECP) = $\dfrac{ATP + 0.5xADP}{ATP + ADP + AMP}$

Experiment 5 : Effect of chronic administration on the

2-deoxy-D-glucose uptake into rat brain Compound A was administered intraperitoneally to male Wistar-rat once daily for 14 days. $^3$H-2-deoxy-D-glucose (40 µCi/kg, i.p.) was injected to rats 27 hours after the last administration of Compound A. Fifteen minuts after the injection of $^3$H-2-deoxy-D-glucose, the animals were decapitated and blood from trunk was collected simultaneously. The brains were dissected into five regions. Radioactivity in the brain sample and serum was counted using Soluene-350 and toluene scintillator. The results are summerized in Table 9. Each value represents the mean ± standard error.

Table 9 : Effect of chronic administration on the

2-deoxy-D-glucose uptake into various regions

of rat brain.

| Region | Dose (mg/kg) | (n) | $\dfrac{\text{dpm/mg tissue}}{\text{dpm/µl serum}}$ (Δ%) |
|---|---|---|---|
| Parietal cortex | 10 | 5 | 1.27 ± 0.11* (+21) |
| Hippocampus | 10 | 5 | 0.90 ± 0.07* (+20) |

* p < 0.05 : significant difference vs. control

Experiment 6 : Acute Toxicity

Compound A was orally administered to male Wistar-rats weighing about 200 g and male ddY-mice weighing 20-25 g. After one week-observation, the $LD_{50}$ value (mg/kg) was measured. The results are shown in Table 10.

Table 10 : Acute toxicity in rats and mice.

| Animals | $LD_{50}$ (mg/kg, p.o.) |
|---------|------------------------|
| Rats | 1,712 |
| Mice | 716 |

The results of above experiments show that the compound of the present invention exhibits really remarkable activity, and is useful as an active ingredient of the drug for improvement and treatment of various diseases associated with cerebrovascular damages or aging.

The compound of the present invention can be administered orally or parenterally, i,e, intraperitoneally, intraveneously or subcutaneously, in human beings in the form of a pharmaceutical composition such as tablets, sugar-coated tablets, powder, granules, pills, syrup, injectable solution or suppositories.

The pharmaceutical composition can prepared by, for example, mixing a therapeutically effective amount of the compound of the present invention with a conventional and pharmaceutically acceptable additives such as an excipient, an extender on a diluent.

The choice of such additive is determined by the preferred form of administration, the solubility of the compound and standard pharmaceutical practice.

The dose may vary depending upon the disease to be treated or the conditions of the patients to be treated, but the daily dose for human adults preferably ranges from 10 to 300 mg, in one to several times divided doses.

Formulation Example : 20.0 mg tablets can be prepared according to the following composition

| | |
|---|---|
| Compound A | 20.0 mg |
| Crystalline cellulose | 10.0 |
| Lactose | 44.5 |
| Corn starch | 15.0 |
| Magnesium stearate | 0.5 |
| | 90.0 mg |

Compound A (20.0 mg), crystalline cellulose (10.0 mg) and lactose (44.5 mg) are mixed well and kneaded with a binder prepared by corn starch (1.0 mg), and then the reminder of corn starch and magnesium stearete (0.5 mg) are admixed and compressed into tablets weighting 90.0 mg.

0193637

What is claimed is :

1.    A use of 2-(2-(2-thienylmethyl)phenoxymethyl)morpholine or a pharmaceutically acceptable acid addition salt thereof for the manufacture of a medicament for the improvement or treatment of various diseases associated with cerebrovascular damages or aging.

2.    The use of 2-(2-(2-thienylmethyl)phenoxymethyl)morpholine maleate for the manufacture of a medicament for the improvement or treatment of various disease associated with cerebrovascular

damages or aging.

0193637

European Patent Office

**EUROPEAN SEARCH REPORT**

Application number

EP 85 10 2670

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 84, no. 7, 16th February 1976, page 499, no. 44084u, Columbus, Ohio, US; & JP - A - 75 89 380 (YOSHITOMI PHARMACEUTICAL INDUSTRIES LTD.) 17-07-1976 * Abstract * | 1,2 | A 61 K 31/535 |
| Y | CHEMICAL ABSTRACTS, vol. 88, no. 2, 9th January 1978, page 300, no. 11910q, Columbus, Ohio, US; & JP - A - 77 87 242 (YOSHITOMI PHARMACEUTICAL INDUSTRIES LTD.) 20-07-1977 * Abstract * | 1,2 | |
| D,Y | US-A-4 005 084 (T. MURO) * Column 1; claims * | 1,2 | |

TECHNICAL FIELDS SEARCHED (Int. Cl.4)

A 61 K 31/00
C 07 D 413/00

The present search report has been drawn up for all claims

| Place of search | Date of completion of the search | Examiner |
|---|---|---|
| THE HAGUE | 31-10-1985 | CREMERS K. |